# EUROPÄISCHE PATENTSCHRIFT

(11) **EP 1 903 033 B1**
(45) Veröffentlichungstag und Bekanntmachung des Hinweises auf die Patenterteilung: **29.04.2009**
(21) Anmeldenummer: 08000437.7
(22) Anmeldetag: 06.09.2002
(51) Int. Cl.: C07D 207/38, A61K 31/4015, A61P 25/28

(54) **Coniosetin, Verfahren zu seiner Herstellung und Verwendung als Arzneimittel**
Coniosetine, process for preparation and use as medicament
Coniosetine, procédé de préparation et utilisation comme médicament

(30) Priorität: 22.09.2001 DE 10146737
(43) Veröffentlichungstag der Anmeldung: 26.03.2008
(62) Teilanmeldung aus: 02777014.8
(73) Patentinhaber: Sanofi-Aventis Deutschland GmbH, 65929 Frankfurt am Main (DE)
(72) Erfinder: Vértesy, László, Dr., 65926 Frankfurt am Main (DE); Ehrlich, Klaus, Dl., 65926 Frankfurt am Main (DE); Kurz, Michael, Dr., 65926 Frankfurt am Main (DE); Segeth, Marian Paul, 65926 Frankfurt am Main (DE); Toti, Luigi, Dr., 65926 Frankfurt am Main (DE)

(56) Entgegenhaltungen:
- WO-A-01/59104
- WO-A-98/21327
- FR-A- 2 740 454
- US-A- 5 420 155
- FASSBENDER K ET AL: "Alzheimer's disease: molecular concepts and therapeutic targets." DIE NATURWISSENSCHAFTEN. GERMANY JUN 2001, Bd. 88, Nr. 6, Juni 2001 (2001-06), Seiten 261-267, XP002243958 ISSN: 0028-1042
- CUTLER N R ET AL: "Review of the next generation of Alzheimer's disease therapeutics: challenges for drug development." PROGRESS IN NEURO-PSYCHOPHARMACOLOGY & BIOLOGICAL PSYCHIATRY. ENGLAND JAN 2001, Bd. 25, Nr. 1, Januar 2001 (2001-01), Seiten 27-57, XP002243957 ISSN: 0278-5846

## Beschreibung

Die Alzheimer'sche Krankheit (Demenz vom Alzheimer-Typ, AD) ist nach heutigem Kenntnisstand eine erbliche, unaufhaltsam fortschreitende Großhirnrinden-Atrophie mit zunehmenden Denkstörungen. Bei der Alzheimer-Krankheit handelt es sich um eine neuropsychiatrische Erkrankung, die vorwiegend bei älteren Menschen auftritt. Die Erkrankung manifestiert sich in einem Symptomenkomplex, die Gedächtnisstörungen, herabgesetzte Merkfähigkeit, Orientierungsstörungen, Sprachstörungen, Störungen des gezielten Denken usw. einschließt. Bei Alzheimer-Patienten findet man charakteristische neurohistologische Veränderungen, wie beispielsweise die Ablagerungen sogenannter amyloider Plaques sowie auch eine Degeneration der Neurofibrillen in den Nervenzellen (sog. "fibrilläre Bündel"). Diese Veränderungen sind zwar charakteristisch, jedoch keineswegs spezifisch, da sie in geringerem Ausmaß auch beim normalen Alterungsprozess auftreten.

Derzeit ist keine kausale, sondern nur eine symptomatische Behandlung der AD möglich. Bisher gibt es Medikamente, die den Verlauf der Krankheit lediglich verzögern, sie aber nicht heilen können. Den wichtigsten therapeutischen Ansatz bietet die Gruppe der cerebralen Acetylcholinesterase-Hemmer (Tacrin®, Donepezil®, Rivastigmin®, Galantamin®), da für die gedächtnisrelevanten Strukturen, die bei AD in besonderem Maße beeinträchtigt sind, die cholinerge Signalübertragung eine große Rolle spielt. Die Medikamente können allerdings nur bei leichtem und mittlerem Krankheitsstadium eingesetzt werden. Sie erhöhen die Konzentration von Acetylcholin in den informationsübertragenden Synapsen des Gehirns. Bei zu starker Schädigung der Neuronen, also im Spätstadium der Krankheit, sind sie nicht mehr wirksam. Weitere Substanzen, deren Einsatz überprüft wird, sind Östrogene, nicht-steroidale Analgetika, Antioxidantien und Nerven-Wachstumsfaktoren (NGF). Alle diese Mittel sind aber in ihrer Wirkung zur Behandlung der Alzheimer'schen Erkrankung unzulänglich.

Man schätzt, dass es gegenwärtig etwa eine Million Menschen in der Bundesrepublik Deutschland gibt, die an der Alzheimer'schen Krankheit leiden. Diese Zahl wird vermutlich aufgrund der steigenden Lebenserwartung der Bevölkerung in den nächsten Jahren noch weiter ansteigen (F. Kohl, Prax. Naturwiss. Biol. (1999), 48(6), 26-31). Daher sind dringend neue Substanzen zur Behandlung dieser Erkrankung notwendig.

Die für die AD charakteristischen histologischen Gehirnveränderungen sind die sog. Amyloid-Plaques. Es sind krankhafte Proteinablagerungen des β-Amyloid-Peptids bzw. des ßA4-Proteins, welches, infolge eines Stoffwechseldefektes, aus einem physiologischen Zellmembran-Bestandteil, dem Amyloid Precursor Protein (APP), heraus gespalten wird. Es reichert sich dann innerhalb des Gehirns an, wo es vom Körper nicht mehr abgebaut werden kann und als Plaque erscheint (F. Kohl, Prax. Naturwiss. Biol. (1999), 48(6), 26-31; D. J. Selkoe; Trends Cell Biol. (1998), 8, 447-453). Das wesentliche Element des Amyloid-Plaques ist ein 39 bis 43 Aminosäuren umfassendes Peptid, das sogenannte β-Amyloid oder Aβ. Dieses Peptid entsteht bei der Prozessierung des APP. Bei Patienten, die unter der erblichen Form der AD leiden, sind Mutationen in den Genen, die für dieses Prozessieren kodieren, gefunden worden. In denselben Fällen wurde eine Zunahme der Aß-Produktion beobachtet, woraus geschlossen worden ist, daß das Prozessieren des APP ein geeigneter Angriffsort für die Behandlung der Alzheimer'schen Erkrankung sein könnte. Beim Prozessieren des APP spielt die Tetrapeptid-Teilsequenz Asn-Pro-Thr-Tyr eine Rolle. Diese Teilsequenz wird von den Proteinen COFE65 und besonders FE65 erkannt und es wird angenommen, daß die für die APP-Prozessierung verantwortliche Protein-Protein - Wechselwirkung mit ihrer Beteiligung stattfindet (WO 98/21327). Agentien, die die APP - FE65 - Wechselwirkung hemmen, sollten daher wertvolle Mittel zur Behandlung von AD sein.

FR2740454 beschreibt Peptidfragmente des FE65 und ihre Klonierung. Weiterhin beschreibt diese Publikation die Fähigkeit genannter Peptide zur Modulation und letztlich Inhibition von APP - FE65- Wechselwirkung.

Es sind bereits einige Verbindungen mit Farnesylthiopeptid-Grundstruktur bekannt.

Eng Wui Tan et al. (J. Am. Chem. Soc. (1991), 113, 6299-6300) und Bryant A. Gilbert (J. Am. Chem. Soc. (1992), 114, 3966-3973) beschreiben die Verbindungen Farnesylcystein und Farnesylcysteinoxid, 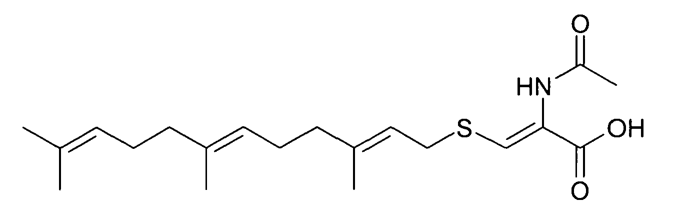 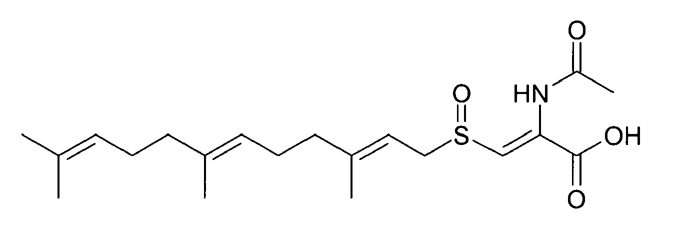 die gute Substrate der isoprenylierten Proteinmethyltransferase sind.

Zuvor war schon die Bedeutung der Prenylierung von Proteinen für deren Verankerung in biologische Membranen bekannt (J. A. Glomset et al., Trends in Biochem. Sciences (1990), 15, 139-142).

Miyakawa et al. (J. Bacteriol. (1982), 151, 1184-1194) beschreiben die Verbindungen Rhodotorucine A (p=0) und Rhodotorucine-A-S-oxid (p=1), 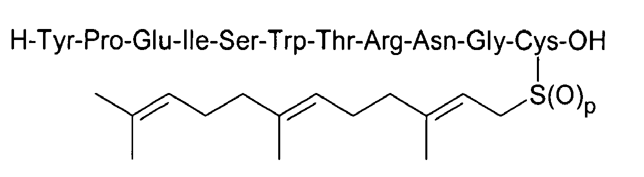 als *Rhodosporidium toruloides* Typ-A-Zell Sexualhormone.

Es wurde überraschend gefunden, daß der Stamm *Coniochaeta ellipsoidea* Udagawa, DSM 13856, neue Verbindungen zu bilden vermag, welche nicht nur die APP-FE65-Wechselwirkung wirksam hemmen, sondern auch gut verträglich sind.

Der Pilz *Coniochaeta ellipsoidea* Udagawa wurde bei der Deutschen Sammlung von Mikroorganismen und Zellkulturen GmbH (DSM), Mascheroder Weg 1 B, 38124 Braunschweig, Deutschland nach den Regeln des Budapester Vertrages am 17.11.2000 unter der folgenden Nummer hinterlegt: DSM 13856. Der Pilz besitzt ein weisses Substratmycel und ganz wenig Luftmycel und bildet in Kultur keine für Coniochaeta charakteristischen Fruchtkörper.

Anstelle des Stammes DSM 13856 können auch deren Mutanten und/oder Varianten eingesetzt werden, soweit sie die besagten Verbindungen synthetisieren.

Mutanten sind zur selben Species gehörenden Organismen, die sich in ihren Genen unterscheiden und damit verschiedene Genotypen darstellen ("Genotype: The genetic constitution of an organism, usually in respect to one gene or a few genes relevant in a particular context", McGraw-Hill, Dictionary of scientific and technical terms, McGraw-Hill Book Company, N. Y. 1978, Seite 672).

Die Herstellung von Mutanten ist unter anderem beschrieben in Brock et al. "Biology of Microorganisms", Prentice Hall, S. 238-247 (1994), wonach beispielsweise physikalische Mittel, z.B. Bestrahlung mit Ultraviolett- oder Röntgenstrahlen, oder durch chemische Mutagene, wie z.B. Ethylmethansulfonat (EMS); 2-Hydroxy-4-methoxy-benzophenon (MOB) oder N-Methyl-N'-nitro-N-nitrosoguanidin (MNNG) Mutationen induzieren können.

Varianten im Sinne der Beschreibung unterscheiden sich bei identischem Genom im Phenotyp vom Wildtyp ("Phenotype: The observable characters of an organism", McGraw-Hill, Dictionary of scientific and technical terms, McGraw-Hill Book Company, N. Y. 1978, Seite 1199). Mikroorganismen haben die Fähigkeit, in Abhänigigkeit von ihrer Umgebung verschiedenen Phenotypen auszubilden: "Microorganisms have the ability to adapt to enviromental changes. This adaptive capacity is the reason for the observed physiological flexibility. In phenotypic adaptation, all cells of a population are involved. This type of change is not genetically conditioned. It is a modification that under altered conditions is reversible" (H. Stolp, "Microbial ecology: organisms, habitats, activities", Cambridge University Press, Cambridge, GB, 1988, Seite 180).

Das Screening nach Mutanten und Varianten, die die besagten Verbindungen produzieren, kann durch Bestimmung der biologischen Aktivität des in der Kulturbrühe angehäuften Wirkstoffes, oder durch Hochleistungsflüssigkeits-Chromatographie (HPLC-Bestimmung) erfolgen.

Als Kohlenstoffquellen für die Fermentation eignen sich beispielsweise assimilierbare Kohlenhydrate und Zuckeralkohole, wie Glukose, Laktose, Saccharose oder D-Mannit sowie kohlenhydrathaltige Naturprodukte, wie z.B. Malzextrakt oder Hefextrakt. Als stickstoffhaltige Nährstoffe kommen beispielsweise Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide in Betracht. Als anorganische Salze kann die Nährlösung beispielsweise Chloride, Carbonate, Sulfate oder Phosphate der Alkali- oder Erdalkalimetalle, enthalten, und als Spurenelemente beispielweise Eisen, Zink, Kobalt, Molybden, Bor, Vanadium und Mangan.

Bevorzugt enthält das Kulturmedium Malzextrakt, Hefeextrakt, Glukose, Stärke, Haferflocken und/oder Glycerin, besonders bevorzugt in Mengenanteilen von 0,05 bis 5 %, bevorzugt 1 bis 2 % Malzextrakt, 0,05 bis 3 %, bevorzugt 0,05 bis 1 % Hefeextrakt, 0,2 bis 5 %, bevorzugt 0,5 bis 2 % Glucose und 0,5 bis 3 %, bevorzugt 1.5% bis 3 % Haferflocken, jeweils bezogen auf das Gewicht der gesamten Nährlösung.

Die Kultivierung des Mikroorganismus erfolgt bevorzugt aerob, also beispielsweise submers unter Schütteln oder Rühren in Schüttelkolben oder Fermentern oder auf Festmedium, gegebenenfalls unter Einführen von Luft oder Sauerstoff. Die Kultivierung kann in einem Temperaturbereich von etwa 15 bis 35°C, vorzugsweise bei etwa 20 bis 35°C, insbesonders bei 25 bis 30°C durchgeführt werden. Der pH-Bereich der Kultivierung kann zwischen 3 und 10 liegen, vorzugsweise zwischen 6.5 und 7.5. Man kultiviert den Mikroorganismus unter diesen Bedingungen im allgemeinen über einen Zeitraum von 48 bis 960 Stunden, vorzugsweise 72 bis 720 Stunden. Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10-1:100, überimpft werden. Die Vorkultur erhält man z.B., indem man das Mycel in eine Nährlösung überimpft und etwa 20 bis 120 Stunden, bevorzugt 48 bis 72 Stunden, wachsen läßt. Das Mycel kann beispielsweise erhalten werden, indem man den Stamm etwa 1 bis 40 Tage, bevorzugt 21 bis 35 Tage, auf einem festen oder flüssigen Nährboden, beispielsweise Hefe-Malz-Agar, Haferflocken-Agar oder Kartoffeldextrose-Agar, wachsen läßt.

Der Fermentationsverlauf und die Bildung der besagten Verbindungen kann entsprechend dem Fachmann bekannten Methoden, wie z.B. durch Testen der biologischen Aktivität in Bioassays oder durch chromatographische Methoden wie Dünnschichtchromatographie (DC) oder Hochleistungsflüssigkeits-Chromatographie (HPLC) verfolgt werden.

Der Stamm *Coniochaeta ellipsoidea* Udagawa, DSM 13856, produziert darüberhinaus ein Coniosetin genanntes Tetramsäure-Derivat der Formel (VI), 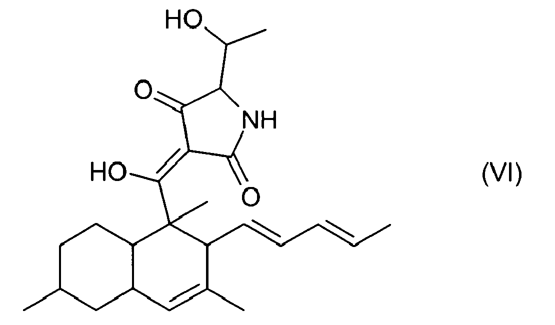 das ebenfalls die APP-FE65-Wechselwirkung hemmt.

Chiralitätszentren in der Verbindung der Formel (VI) können, wenn nicht anders angegeben, in der R- oder in der S-Konfiguration, als optisch reine Verbindungen oder als Stereoisomerengemische, wie Enantiomerengemische und Diasteromerengemische vorliegen.

Vorzugsweise besitzt die Verbindung der Formel (VI) die in Formel (Vl A) genannte Stereochemie:

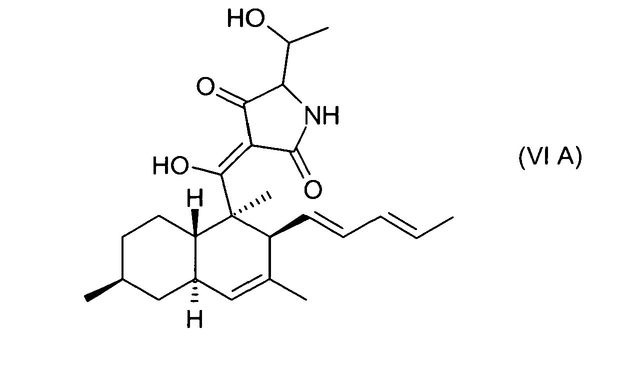

Zur Gewinnnung von Coniosetin der Formel (VI) wird *Coniochaeta ellipsoidea* Udagawa, DSM 13856, fermentiert, wobei die Fermentationstemperatur, der pH-Wert und die Bestandteile des Kulturmediums mit den für die Gewinnung von Coniosulfid und Coniosulfidderivaten genannten Angaben übereinstimmen, anschliessend extrahiert und isoliert. Coniosetin der Formel (VI) kann anschließend optional in ein offensichtliches chemisches Äquivalent und/oder pharmakologisch verträgliches Salz überführt werden.

Offensichtliche chemische Äquivalente der Verbindungen der Formeln (VI) und (Vl A) sind Verbindungen, die einen geringfügigen chemischen Unterschied aufweisen, also die gleiche Wirksamkeit haben oder sich unter milden Bedingungen in die erfindungsgemäßen Verbindungen umwandeln. Zu den genannten Äquivalenten gehören z.B. Ester, Azomethine (Schiff'sche Basen), Hydrierungsprodukte, Reduktionsprodukte, Komplexe oder Addukte der bzw. mit den erfindungsgemäßen Verbindungen, die nach in der Literatur bekannten Methoden hergestellt werden (J. March, Advanced Organic Synthesis, 4th Edition, John Wiley & Sons, 1992).

Doppelbindungen in der Alkylkette in Verbindungen der Formeln (VI) und (VI A) können beispielsweise durch Hydrogenolyse oder nach anderen an sich bekannte Methoden reduziert werden, zum Beispiel beschrieben von P. N. Rylander in "Hydrogenation Methods", Academic Press, New York (1985), Chapter 2, oder von H.O. House in "Modern Synthetic Reactions", W.A. Benjymin, Inc., New York (1972), Seite 446-452. Die Doppelbindungen können außerdem zu Epoxiden oxidiert werden, beispielsweise mittels meta-Chlorperbenzoesäure (MCPBA; J. March, Advanced Organic Synthesis, 4th Edition, John Wiley & Sons, 1992).

Die Verbindungen der Formeln (VI) und (VI A) sowie die offensichtlichen chemischen Äquivalente derselben können nach dem Fachmann bekannten Methoden in die entsprechenden physiologisch verträglichen Salze übergeführt werden.

Unter physiologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z.B. HCl, HBr, H₂SO₄, Maleinsäure, Fumarsäure in Frage.

Coniosetin und Coniosetin-Derivate, ein Verfahren zu deren Herstellung durch Fermentation von *Coniochaeta ellipsoidea* Udagawa, DSM 13856, und die Verwendung von Coniosetin und -derivaten als Arzneimittel sind beschrieben in der deutschen Patentanmeldung Nummer DE 10060810.8.

Es wurde gefunden, daß die Verbindungen der Formeln (VI) und (VI A) starke Hemmung der Amyloid Precursor Protein - FE65 - Wechselwirkung aufweisen, sie eignen sich daher zur Behandlung und Prophylaxe von degenerativen Neuropathien und der Alzheimer'schen Krankheit. Die Hemmung der Amyloid Precursor Protein - FE65 - Wechselwirkung durch Coniosetin beträgt: lC₅₀ = 28 µM.

Ein Gegenstand der Erfindung ist demnach die Verwendung einer Verbindung der Formel (VI), vorzugsweise der Formel (VI A), zur Herstellung von Arzneimitteln zur Behandlung und/oder zur Prophylaxe der Alzheimer'schen Krankheit.

Das besagte Arzneimittel wird durch Mischung von mindestens einer Verbindung der Formel (Vl) mit mindestens einem physiologischen geeigneten Träger und/oder Hilfsstoff hergestellt und in eine geeignete Darreichungsform gebracht.

Die besagten Arzneimittel können enteral (oral), parenteral (intramuskulär oder intravenös), rektal oder lokal (topisch) angewendet werden. Sie können in Form von Lösungen, Pulvern, Tabletten, Kapseln einschließlich Mikrokapseln, Salben, Cremes, Gel, oder Suppositorien verabreicht werden. Als physiologisch geeignete Träger oder Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die beispielsweise die wirksame Tagesmenge der besagten Verbindungen, z.B. 30 - 3000, vorzugsweise 50 - 1000 mg enthalten.

Die folgenden Beispiele sollen der näheren Erläuterung der Erfindung dienen.

### Beispiel 1 Herstellung einer Glycerinkultur von Coniochaeta ellipsoidea, DSM 13856

30 mL Nährlösung (Malzextrakt 2,0%, Hefeextrakt 0,2%, Glucose 1,0 % (NH₄)₂HPO₄ 0,05 %, pH 6,0) in einem sterilen 100 mL Erlenmeyerkolben werden mit dem Stamm *Coniochaeta ellipsoidea* Udagawa, DSM 13856, beimpft und 6 Tage bei 25 °C und 140 UpM auf einer rotierenden Schüttelmaschine inkubiert. 1,5 ml dieser Kultur werden anschließend mit 2,5 ml 80 %igem Glycerin verdünnt und bei -135°C gelagert.

### Beispiel 2 Herstellung einer Vorkultur im Erlenmeyerkolben von Coniochaeta ellipsoidea Udagawa, DSM 13856

100 mL Nährlösung (Malzextrakt 2,0 %, Hefeextract 0,2 %, Glucose 1,0 % (NH₄)₂HPO₄ 0,05 %, pH 6,0) in einem sterilen 300 mL Erlenmeyerkolben werden mit dem Stamm *Coniochaeta ellipsoidea* Udagawa, DSM 13856, beimpft und 4 Tage bei 25 °C und 140 UpM auf einer rotierenden Schüttelmaschine inkubiert. 2 ml dieser Vorkultur werden anschließend für die Herstellung der Hauptkulturen verwendet.

### Beispiel 3: Herstellung einer Hauptkultur von Coniochaeta ellipsoidea Udagawa , DSM 13856, auf Festmedium Platten.

30 sterile 22 x 22 cm Platten werden mit 200 ml einer Nährlösung enthaltend 20 g/l Malzextrakt, 20 g/l Haferflocken, 2% Agar und eines pH Wertes 7.0 gegossen. Diese Platten werden mit 2 ml einer Vorkultur von *Coniochaeta ellipsoidea* Udagawa , DSM 13856, gewonnen nach Beispiel 2, beimpft und bei 25° C inkubiert. Die maximale Produktion einer oder mehrerer Verbindungen des erfindungsgemäßen Coniosetins ist nach ca. 676 Stunden erreicht.

### Beispiel 4: Isolierung von Coniosetin.

30 Agarplatten, Größe je 25 x 25 cm, gewonnen nach Beispiel 3, werden gefriergetrocknet und mit 2.5 Liter Methanol extrahiert. Die klare flüssige Phase wird im Vakuum auf 100 mL eingeengt, mit Wasser verdünnt und auf eine 580 mL fassende, mit dem Adsorptionsharz MCI Gel® CHP20P gefüllte Säule aufgetragen. Säulenmaße: Breite x Höhe: 5 cm x 30 cm. Eluiert wird mit einem Lösungsmittel-Gradienten von 5 % Acetonitril in Wasser bis 90 % Acetonitril und der Säulenausfluß (40 mL/Minute) in Fraktionen je 120 mL aufgefangen. Die Coniosetin-haltigen Fraktionen, die durch HPLC-Analysen überprüft werden, werden gesammelt und im Vakuum konzentriert sowie gefriergetrocknet (0.3 g).

### Beispiel 5: Hochdruckflüssigkeitschromatographie (HPLC) des Coniosetins.

| | |
|---|---|
| Säule: | Superspher 100 RP-18e®, 250-4, mit Vorsäule, |
| Mobile Phase: | 75 % Acetonitril in 0.1% Phosphorsäure, |
| Flußgeschwindigkeit: | 1 mL pro Minute, |
| Detektion durch UV-Absorption bei 210 nm. | |

Die Retentionszeit von Coniosetin beträgt 13.6 Minuten.

### Beispiel 6: Endreinigung des Coniosetins.

Das angereicherte Antibiotikum Coniosetin (0.3 g), gewonnen nach Beispiel 4, wird auf einer LiChrospher® 100 RP-18e-HPLC-Säule (Breite x Höhe = 2.5 cm x 25 cm) im Gradientenverfahren mit 75 % bis 100 % Acetonitril in 0,05 % Essigsäure aufge-trennt. Fluß: 30 mL/Min. Fraktionsgröße: 60 mL. Die durch analytische HPLC (siehe Beispiel 5) untersuchten Fraktionen werden entsprechend ihrem Coniosetin-Gehalt zusammengefaßt, im Vakuum eingeengt und gefriergetrocknet. Sie ergeben 170 mg Coniosetin in 98 %iger Reinheit.

### Beispiel 7: Charakterisierung des Coniosetins.

### Bestimmung des Molpeaks:

Dem gesuchten Molekül wird die Masse 413 zugeordnet aufgrund folgender Befunde: ESI⁺-Spektrum sowie FAB⁺-Spektren zeigen Peaks bei 414 amu (M+H)⁺. ESI⁻ - Spektrum zeigt unter anderem einen Peak bei 412 amu (M-H)-.

### Hochauflösung des Quasi-Molekülions:

Unter FAB-Bedingungen mit einer Nitrobenzylalkohol-Matrix wird u.a. ein Peak bei 414.2645 amu beobachtet. Die bei der Messung vorliegenden Massen-genauigkeit beträgt ca. 5 ppm. Der Meßwert stimmt gut mit dem für C₂₅H₃₆NO₄ = 414.2644 amu berechneten Elementarzusammensetzung überein. Bei dieser Elementarzusammensetzung liegen 9 Doppelbindungsäquivalente vor.

Die physikalisch-chemischen sowie spektroskopischen Eigenschaften des erfindungsgemäßen Antibiotikums lassen sich wie folgt zusammenfassen:

### Coniosetin:

### Aussehen:

Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.

| | |
|---|---|
| Summenformel: | C₂₅H₃₅NO₄ |
| Molekulargewicht: | 413,56 |

¹H- und ¹³C-NMR: siehe Tabelle 6 und 7
UV-Maxima: 233 nm, 288 nm

**Tabelle 6: ¹H- und ¹³C- chemische Verschiebungen von Coniosetin in DMSO-d₆ und Methanol-d₄ bei 300K.**

| | DMSO-d₆ | | Methanol-d₄ | |
|---|---|---|---|---|
| Position | ¹³C | ¹H | ¹³C | ¹H |
| | δ (ppm) | δ (ppm) | δ (ppm) | δ (ppm) |
| 1 | 48.94 | - | 51.18 | - |
| 1-Me | 13.34 | 1.33s | 14.31 | 1.42 s, br |
| 2 | 48.45 | 3.19 | 50.59 | 3.28 br |
| 3 | 130.99 | - | 133.15 | - |
| 3-Me | 22.04 | 1.53 t | 22.67 | 1.58 t |
| 4 | 125.88 | 5.19 s, br | 127.31 | 5.20 s |
| 5 | 38.61 | 1.80 | 40.68 | 1.86 m |
| 6 | 42.06 | 1.78, 0.82 | 44.10 | 1.83 d, br, 0.87 m |
| 7 | 32.90 | 1.49 | 35.00 | 1.52 m, br |
| 7-Me | 22.40 | 0.89 d | 23.07 | 0.94 d |
| 8 | 35.44 | 1.72, 1.01 | 37.20 | 1.77 d, br, 1.10 m |
| 9 | 27.59 | 1.94 d, 1.00 | 29.47 | 2.01 d, br, 1.06 m |
| 10 | 39.28 | 1.57 | 41.39 | 1.66 m |
| 11 | 130.42 | 5.18 m | 132.05 | 5.19 |
| 12 | 131.96 | 5.72 t | 134.03 | 5.78 t |
| 13 | 131.31 | 5.91 t | 132.71 | 5.90 t |
| 13a | 127.83 | 5.52 m | 129.12 | 5.51 m |
| 13b | 17.73 | 1.65 d | 18.23 | 1.67 d |
| 14 | 198.23 | - | 201.30 | - |
| 14-OH | - | 17.49 s, br | - | - |
| 15 | 99.46 | - | 101.50 | - |
| 16 | 179.52 | - | 181.53 | - |
| 17 | - | 9.22 s, br | - | - |
| 18 | 66.57 | 3.62 | 68.19 | 3.62 br |
| 19 | 191.09 | - | 193.51 | - |
| 20 | 65.66 | 3.91 | 68.11 | 4.06 br |
| 20-OH | - | 4.76 d | - | - |
| 21 | 20.67 | 1.17 d | 20.65 | 1.29 d, br |

**Tabelle 7: ¹H- und ¹³C- chemische Verschiebungen von Coniosetin in CDCl₃ bei 300K.**

| Position | ¹³C | ¹H |
|---|---|---|
| | δ (ppm) | δ (ppm) |
| 1 | 49.84 | - |
| 1-Me | 13.76 | 1.44 |
| 2 | 49.25 | 3.22 |
| 3 | 131.50 | - |
| 3-Me | 22.23 | 1.61 |
| 4 | 126.01 | 5.20 |
| 5 | 39.13 | 1.85 |
| 6 | 42.54 | 1.82, 0.90 |
| 7 | 33.54 | 1.54 |
| 7-Me | 22.46 | 0.94 |
| 8 | 35.78 | 1.79, 1.13 |
| 9 | 28.30 | 1.99, 1.08 |
| 10 | 39.73 | 1.68 |
| 11 | 130.19 | 5.23 |
| 12 | 132.62 | 5.83 |
| 13 | 131.39 | 5.89 |
| 13a | 128.19 | 5.52 |
| 13b | 18.02 | 1.70 |
| 14 | 200.23 | - |
| 14-OH | - | - |
| 15 | 100.28 | - |
| 16 | 179.33 | - |
| 17 | - | 6.19 |
| 18 | 65.43 | 3.71 |
| 19 | 190.95 | - |
| 20 | 67.80 | 4.05 |
| 20-OH | - | - |
| 21 | 19.56 | 1.34 |

### Beispiel 8: Hemm-Test der APP - FE65 - Wechselwirkung.

Reagenzien und Bezugsquellen:

| | |
|---|---|
| HEPES, | Sigma, H-9136, |
| NaCl, | Riedel de Haen, 31434, |
| EDTA, | Sigma, E-1644, |
| CHAPS, | Sigma, C-5849, |
| KF, | Sigma, P-1179, |
| BSA, | Sigma, A-9647 |
| Monoclonale Anti GST Antikörper | |
| markiert mit XL665, | CIS bio Intern., D11/459/12/47270; |
| Streptavidin markiert mit Eu-cryptat, | CIS bio Intern., D11/450/12/47269; |
| 32mer cAPP, | Sigma, E2533; |
| Biotin-cAPP, | Aventis SA, Vitry. |

### Puffer A:

10 mM HEPES, pH 7.2 + 150 mM NaCl + 3.4 mM EDTA + 0.184 g/L CHAPS.

### Puffer B:

100 mM HEPES, pH 7.0 + 400 mM KF + 133 mM EDTA + 1 g/L BSA.

### Durchführung:

1 µl der zu prüfenden Lösung wurde zusammen mit 30 µM 32mer cAPP in Puffer A in eine Mikrotiterplatte pipettiert. Dann wurde 1 µl Biotin-cAPP (10nM, gelöst in Puffer A) hinzugefügt. Nach 15 minütigem Inkubieren setzte man 1 µL GST-PTB2 (20 nM in Puffer A) hinzu und inkubierte erneut 15 Minuten lang. Dann wurden 4 µl Antikörpergemisch (Streptavidin markiert mit Eu-cryptat, 2 ng pro well; Monoklonaler Anti GST Antikörper markiert mit XL665, 25 ng in Puffer B) hinzugefügt.
Nach 30 Minuten bei Raumtemperatur wurde in einem Tecan Ultra Photometer das Emissions-Signal der Energie-Übertragung sowie das Europium-Signal gemessen bei 665nm und 615 nm, nachdem die Testlösung mit Licht der Wellenlänge 340 nm angeregt worden ist. Für die Hemmung der Amyloid Precursor Protein - FE65 - Wechselwirkung wurde gefunden: IC₅₀ = 28 µM.

## Patentansprüche

1. Verbindung der Formel (VI) 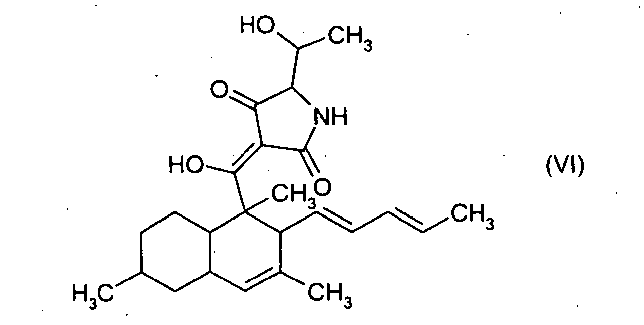 zur Behandlung und Prophylaxe der Alzheimer'schen Krankheit.

2. Verbindung der Formel (VI) gemäß Anspruch 1, **gekennzeichnet durch** die Verbindung der Formel (VI A)

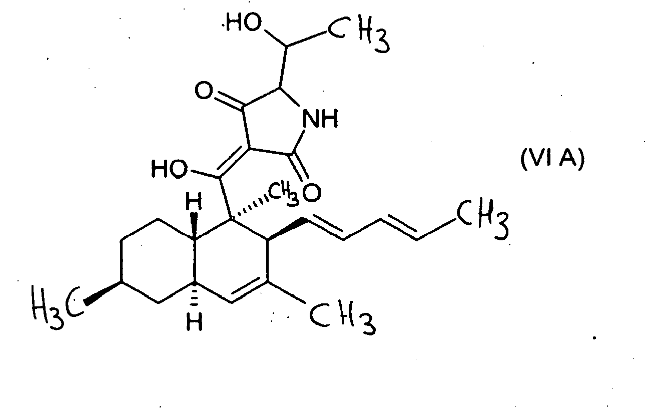

## Claims

1. Compound of formula (VI) 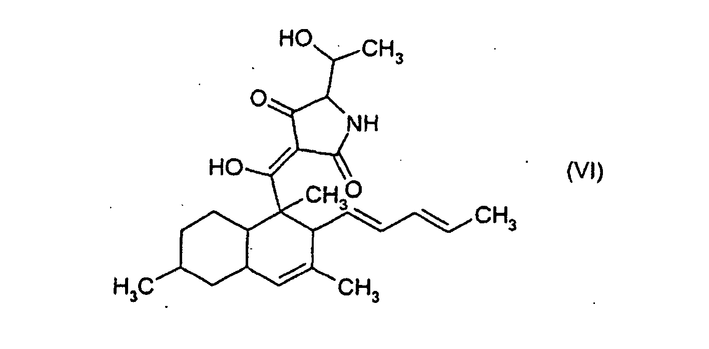 for treatment and prophylaxis of Alzheimer's.

2. Compound of formula (VI) according to claim 1, **characterized by** the compound of formula (VI A)

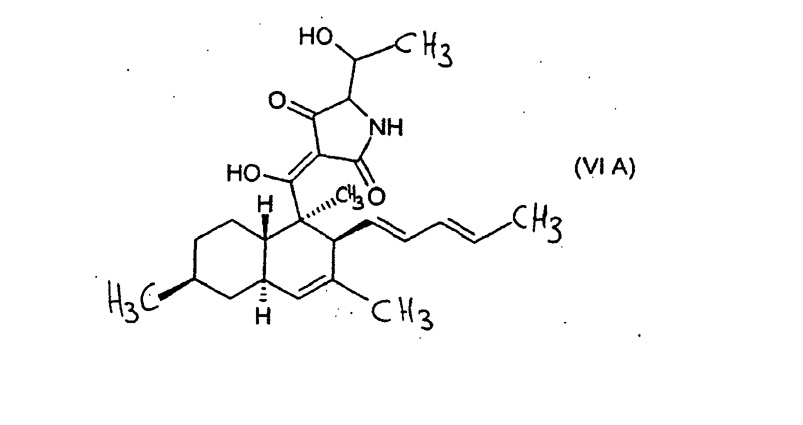

## Revendications

1. Composé de formule (VI) 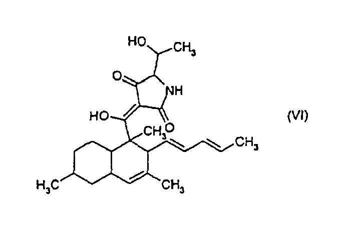 pour le traitement et la prophylaxie de la maladie d'Alzheimer.

2. Composé de formule (VI) selon la revendication 1, **caractérisé par** le composé de formule (VI A)
